# EUROPEAN PATENT APPLICATION

(11) **EP 3 831 364 A2**
(43) Date of publication of application: **09.06.2021**
(21) Application number: 19812837.3
(22) Date of filing: 19.06.2019
(51) Int. Cl.: A61K 9/08, A61J 1/14, A61K 31/4152

(54) **LIQUID DOSAGE FORM OF EDARAVONE OR PHARMACEUTICALLY ACCEPTABLE SALTS THEREOF WHICH IS STABLE IN STORAGE, TRANSPORTATION AND USE**

(30) Priority: 29.08.2018 UA 201808989
(71) Applicant: Sia Emteko Holding, 1046 Riga (LV)
(72) Inventor: HUMENIUK, Mykola, Kyiv, 03110 (UA)
(74) Representative: Godlewski, Piotr
(86) International application number: PCT/IB2019/000611
(87) International publication number: WO 2020/044106

(57) **Abstract**

The invention relates to a method of obtaining a liquid dosage form of an edaravone drug, stable during storage, transportation and convenient for use, for parenteral administration. The method involves preparing a solution containing as active ingredient edaravone or its pharmaceutically acceptable salts and excipients (acid component, alkaline component, antioxidant, osmolar agent and / or stabilizer); packing said dosage form into a pre-sterilized glass vial with a lid covered at least partially by an adhesive coating; closure of the vial with a lid and sterilization the vial containing a solution of edaravone or its pharmaceutically acceptable salts as the active ingredient and excipients. The invention also relates to a method of packing liquid dosage form of edaravone drug, that is stable during storage, transportation and convenient to use for parenteral administration, said method involves: i) sterilization of a glass vial and a lid, spill a solution containing edaravone or its pharmaceutically acceptable salts as the active ingredient and excipients (acid component, alkaline component, antioxidant, osmolar agent and/or stabilizer), into a sterilized glass vial; closure of the vial containing solution with a lid covered at least partially by an anti-adhesive coating; sterilization of the sealed vial containing liquid dosage form. The invention also relates to a vial filled with a liquid dosage form of a medicinal product for parenteral administration containing as active ingredient edaravone or its pharmaceutically acceptable salts, and excipients (acid component, alkaline component, antioxidant, osmolar agent and/or stabilizer).

## Description

The invention relates to the field of chemical and pharmaceutical industry and medicine, in particular to a method of obtaining a drug based on edaravone, that is stable during storage, transportation and use.

It is known that edaravone (1-phenyl-3-methyl-5-pyrazolone (phenylmethylpyrazolone)) is a pyrazolone derivative of the formula C10H10N2O and is used as a drug for lateral amyotropic sclerosis. Edaravone has the ability to absorb free radicals and this is due to its use as an acceptor of free radicals in cerebral infarction.

Edaravon is administered intravenously by infusion or drip.

However, there is a significant problem with the use of edaravone - its instability in solution, in particular the adhesion of molecules of the substance on the surface of the vial lid. In addition, when using insufficiently sealed packaging, the concentration of edaravone in the solution increases due to the evaporation of the liquid.

In order to overcome these problems, special packaging of edaravone is attempted, for instance packing into glass ampoules, to ensure the tightness storage of the solution with unchanged drug concentration.

In order to get rid of residual oxygen in the space above the solution of edaravone and to ensure the stability of the drug, nitrogen is added before welding ampoules, filled with edaravone solution, as described in patent CN101933899, 05.01.2011.

However, the ampoule packaging has several disadvantages. A small fixed volume of the drug does not allow to use the solution continuously (for example, with drip) in the desired volume. There is also a risk that small particles of glass getting into the infusion solution when the ampoule is opened.

As described in patent JP2016022092, 08.02.2016, they try to solve said problem via replacing the glass ampoule with a plastic container. Such packaging usually has a composite structure. The plastic container is formed by an outer polypropylene layer, an intermediate polypropylene layer, and an inner layer of cyclic polyolefin.

A vial, a bag or a syringe pre-filled with a solution of edaravone is also used for the packaging of edaravone. These packages are at least partially made of plastic, in particular of cyclic polyolefin resin (patent JP2009084203, 23.04.2009). Said plastic container is provided with a rubber cap made of elastomer, isoprene rubber or butyl rubber, and the surface which is in contact with the solution of edaravone, covered with fluorine or parylene resin.

However, when plastic is used, there is a problem of tightness, namely, oxygen from the air enters the solution of edaravone. Due to the high affinity edaravone combines with oxygen and changes the properties of the drug. To overcome this problem, antioxidants and double packaging are used to stabilize the edaravone solution, as described in patent JP2011136973, July 14, 2011. That makes the process of obtaining a stable drug more complicate. The container is made of polypropylene, polyethylene or other flexible plastic. It has a rubber stopper of elastomer coated with a fluorine coating. The container is packed into another container having reduced oxygen permeability and made of a film based on aluminum oxide or silicon oxide or other similar material.

Said package provides stability of the drug, however it is not easy to use because after opening or damaging the outer container, it loses its protective property. In addition, such packaging does not provide containers of different sizes (volumes), which can lead to misuse of the drug.

Therefore, a ready-to-use edaravone drug or its pharmaceutically acceptable salts is available in the art. Packaging in the form of ampoules is sealed, but the disadvantage of this form of preparation is the inability to reuse one ampoule and the likelihood of getting into the infusion solution of glass particles. Also, the drug is produced in a larger plastic package, which is not tight enough and, as a result, leaks oxygen from the environment. That negatively affects the quality of the drug.

The multilayer plastic container performs its function of ensuring the stability of the drug during storage and transportation only until it is opened. After opening the container, the drug must be used urgently, as tightness is broken and edaravone is degrading. As a result, the allowable level of impurities in the preparation increases. That can cause undesirable side effects in patients. In addition, this form of packaging is complicate for the production of the preparation itself (plastic must withstand double high heat treatment with the preparation), and for the production of packaging *per se* (double container is made of a triple layer of plastic).

The problem is solved by creating a method for obtaining stable during storage, transportation and convenient for use liquid dosage form of drug for parenteral administration, where the said method involves: i) preparation of a solution containing edaravone or its pharmaceutically acceptable salts as active ingredient and auxiliary substances (acid component, alkaline component, antioxidant, osmolar agent and / or stabilizer); ii) packing said dosage form into a pre-sterilized glass vial with a lid covered at least partially by an anti-adhesive coating; iii) closing of the vial with a lid, that at least partially covered with an anti-adhesive coating and sterilizing the vial filled with solution, comprising an edaravone or its pharmaceutically acceptable salts as the active ingredient and excipients. According to claimed method, the glass vial is made of borosilicate glass, the lid is made of elastic polymer and covered at least partially with anti-adhesive coating of polychlorotrifluoroethylene (PCTFE), perfluoroalkoxy alkane (PFA), ethylene-tetrafluoroethylene (ETFE), fluoroethylene propylene (FEP), perfluoropolyether (PFPE) or polyvinyldene fluoride (PVDF).

The problem is also solved by creating of a method for packaging a liquid dosage form of drug edaravone stable during storage, transportation and convenient for use for parenteral administrating, where said method involves: i) sterilizing of a glass vial with a lid, ii) spilling a solution containing edaravone or its pharmaceutically acceptable salts as an active ingredient and excipients (acid component, alkaline component, antioxidant, osmolar agent and/or stabilizer) in a sterilized glass vial; iii) closing (stopping) the vial with solution with the lid covered of at least partially with anti-adhesive coating; iv) sterilizing of the sealed vial with liquid dosage form. According to said method, the glass vial is made of borosilicate glass, the lid is made of an elastic polymer and at least partially covered with an anti-adhesive coating of polychlorotrifluoroethylene (PCTFE), perfluoroalkoxy alkane (PFA), ethylene-tetrafluoroethylene (ETFE), fluoroethylene propylene (FEP), perfluoropolyether (PFPE) or polyvinyldene fluoride (PVDF).

The problem is also solved by creating a vial filled with a liquid dosage form of a medicinal product for parenteral administration containing as active ingredient edaravone or its pharmaceutically acceptable salts, and excipients (acid component, alkaline component, antioxidant, osmolarizing agent and/or stabilizer). Said vial is made of glass, closed with a lid made of a material based on an elastic polymer, the lid is at least partially covered with an anti-adhesive coating. According to technical solution, the vial is made of borosilicate glass; the lid is made of rubber derivative or thermoplastic and its inner surface or full surface is coated with an anti-adhesive coating of polychlorotrifluoroethylene (PCTFE), perfluoroalkoxy alkane (PFA), ethylene-tetrafluoroethylene (ETFE), fluoroethylene propylene (FEP), perfluoropolyether (PFPE) or polyvinyldene fluoride (PVDF). Also an aluminum cap that squeezes the lid of the vial to seal the container with the solution is used.

This technical solution provides the liquid dosage form of the medicinal product that contains as an active ingredient 1-phenyl-3-methyl-5-pyrazolone (edaravone). Said dosage form also comprises the acid component selected from hydrochloric acid, phosphoric acid, sulfuric acid or citric acid; the alkaline component selected from sodium hydroxide or potassium hydroxide; an antioxidant selected from ascorbic acid, bisulfite sodium, metabisulphite sodium, fumaric acid or malic acid or combination thereof; an osmolarizing agent selected from sodium chloride, sodium bicarbonate, potassium chloride, sorbitol, glucose or mannitol; a stabilizer selected from ethylenediaminetetraacetic acid, a disodium salt of ethylenediaminetetraacetic acid, cysteine, sorbitol, propylene glycol or no any of the above. According to the claimed method, the drug is a solution for injection or infusion.

A preparation containing edaravone or its pharmaceutically acceptable salt and auxiliary components should not be exposed to air, the pH and solution concentration should not be changed. The drug should not be placed in conditions that lead to the formation of other chemical complexes due to the adhesion of part of the drug on the walls of the vial. Then its properties will be maximally preserved. Therefore, it is necessary to create special storage conditions for said preparation.

Edaravone is used by drop infusion for certain indications in large volumes. Therefore, it is important that the drug is in use under conditions that will ensure the stability of its chemical structure and prevent the formation of side compounds in solution.

Also, it must be borne in mind that the packaging of the product requires dual sterilization during preparation, so the materials that used to make the container for the finished solution of edaravone should have the appropriate characteristics.

Compliance with all of these conditions will ensure the stability of the drug, extend the shelf life, make the solution of edaravone safe to use, during storage and transportation.

The packaging material, which is intended to provide solution stability without change of pH, tightness and heat resistance, is borosilicate glass, which easily withstands temperatures above 200 degrees Celsius and does not change the chemical composition of the edaravone solution. Therefore, the solution is placed in a container of borosilicate glass.

The closure of the vial filled with the medicinal product must be carried out in such a way that the lid, first, closes the vial tightly, secondly, the lid must be heat-resistant, and third, the lid must be made of a material that prevents the adhesion of the active substance on its surface.

According to the claimed technical solution, the body of the cover is made of an elastic polymer based on a rubber derivative or thermoplastic, which has high impermeable properties, high heat resistance, strength, as well as good flexibility that prevents the fragility of the cover. Other similar materials that meet these characteristics, for example, from the class of unnatural synthetic elastomers, can also be used.

The surface of the lid, partially or completely, at least part of it that is in contact with the solution of the composition must be covered with a layer of substance with anti-adhesive properties, which does not affect the chemical composition of the mixture and is heat-resistant.

Such substances include fluorine-containing polymers. These are dense and durable materials (eg, polytetrafluoroethylene (PTFE) having a density index of 2.18-2.21 g / cm3 and strengths of 15-27N / mm2) that can be used in the temperature range from -269 to +260 degrees Celsius. These compounds do not chemically bond with other compounds. Even aggressive substances do not affect them at high temperatures. Such materials include polychlorotrifluoroethylene (PCTFE), perfluoroalkoxy alkane (PFA), ethylene tetrafluoroethylene (ETFE), fluoroethylenepropylene (FEP), perfluoropolyether (PFPE), or polyvinyldene fluoride (PVDF). Covering the lid with these compounds can be carried out as described in patent JPS6397173, 27.04.1988.

The additional stability of the preparation in the specified packing during transportation and storage is conditioned by the use of an aluminum cap that squeezes the lid of the vial in order to seal the container with the solution.

The barrier capacity of the packaging to absorb oxygen or other compounds from the atmosphere into the solution of the drug edaravone is enhanced by the materials.

This invention is disclosed in the following examples.

### Example 1.

Studies have shown that when a drug based on edaravone is stored in the above-described package there is no interaction of the solution with the packaging materials. That makes increasing impurities impossible.

A comparative analysis of the drug edaravone properties under storing in different standard packaging for such drugs was carried out (Table 1). Ready-to-use solution containing edaravone as the active substance and excipients such as acid, alkaline components, antioxidant, osmolarizing agent and stabilizer was placed in a polymeric package, borosilicate glass vial, sealed with a rubber lid, a plastic container, manufactured by blow technology-fill-seal and into a borosilicate glass vial with a lid made of isopropylene isoprene rubber coated with a fluorine-containing polymer according to the technical solution. The packaged preparation was kept under the same conditions for 24 months, checking the solutions in different packages was carried out after 3, 6, 12 and at the end of the holding period, after 24 months.

The results showed that packaging of a solution of edaravone using glass is more efficient, and the use of a fluorine-containing polymer to cover the lid in combination with borosilicate glass of the vial extended the shelf life from 6 to 24 months.

### Example 2.

Various variants of the composition (solution) of edaravone were prepared using different types of fluorine-containing substances to cover the lid of the vial containing the drug solution. The options are given in Examples 2.1-2.8.

### Example 2.1

| **Component** | **Content in 1 ml of the drug** |
|---|---|
| Edaravon | 0,3 mg |
| Chloride acid | 1,5 mg |
| Sodium hydroxide | 1,5 mg |
| Cysteine | 0,1 mg |
| Sodium chloride | 8,0 mg |
| Sodium Bisulfite | 0,2 mg |

A package is a borosilicate glass vial, sealed with lid made of a rubber derivative-based material, the lid is coated with ETFE polymer.

### Example 2.2

| **Component** | **Content in 1 ml of the drug** |
|---|---|
| Edaravon | 0,3 mg |
| Sulfate acid | 1,5 mg |
| Sodium hydroxide | 1,5 mg |
| Sodium chloride | 8,5 mg |
| Sodium metabisulfite | 0,2 mg |

The package is a borosilicate glass vial, sealed with the lid made of a thermoplastic, the lid is coated with PCTFE polymer.

### Example 2.3

| **Component** | **Content in 1 ml of the drug** |
|---|---|
| Edaravon | 0,3 mg |
| Phosphoric acid | 1,0 mg |
| Sodium hydroxide | 1,5 mg |
| Sodium chloride | 8,6 mg |
| Sodium metabisulfite | 0,2 mg |

A package is a borosilicate glass vial, sealed with lid made of a rubber derivative-based material, the lid is coated with FEP polymer.

### Example 2.4

| **Component** | **Content in 1 ml of the drug** |
|---|---|
| Edaravon | 0,3 mg |
| Phosphoric acid | 1,0 mg |
| Sodium hydroxide | 1,5 mg |
| Sodium chloride | 8,6 mg |
| Sodium Bisulfite | 0,2 mg |

The package is a borosilicate glass vial, sealed with the lid made of a thermoplastic, the lid is coated with PVDF polymer.

### Example 2.5

| **Component** | **Content in 1 ml of the drug** |
|---|---|
| Edaravon | 0,3 mg |
| Phosphoric acid | 1,0 mg |
| Potassium hydroxide | 1,5 mg |
| Sodium chloride | 8,6 mg |
| Ascorbic acid | 0,1 mg |

A package is a borosilicate glass vial, sealed with lid made of a rubber derivative-based material, the lid is coated with PFA polymer.

### Example 2.6

| **Component** | **Content in 1 ml of the drug** |
|---|---|
| Edaravon | 0,3 mg |
| Citric acid | 1,0 mg |
| Sodium hydroxide | 1,5 mg |
| Sodium chloride | 8,6 mg |
| Sodium metabisulfite | 0,2 mg |

A package is a borosilicate glass vial, sealed with lid made of a rubber derivative-based material, the lid is coated with PTFE polymer.

### Example 2.7

| **Component** | **Content in 1 ml of the drug** |
|---|---|
| Edaravon | 0,3 mg |
| Phosphoric acid | 1,0 mg |
| Sodium hydroxide | 1,5 mg |
| EDTA | 0,15 mg |
| Sodium chloride | 8,6 mg |
| Sodium metabisulfite | 0,2 mg |

The package is a borosilicate glass vial, sealed with the lid made of a thermoplastic, the lid is coated with PFPE polymer.

### Example 2.8

| **Component** | **Content in 1 ml of the drug** |
|---|---|
| Edaravon | 0,3 mg |
| Phosphoric acid | 1,0 mg |
| Sodium hydroxide | 1,5 mg |
| Cysteine | 0,1 mg |
| Sodium chloride | 8,6 mg |
| Sodium metabisulfite | 0,2 mg |

A package is a borosilicate glass vial, sealed with lid made of a rubber derivative-based material, the lid is coated with PVDF polymer.

The solutions prepared according to Examples 2.1-2.8 were packed in pre-sterilized glass vials, the vials were closed with a lid covered with an anti-adhesive coating, the vials with solution were sterilized and kept for a certain period.

Series of test were conducted on the quality and stability of the finished pharma product when stored at 25 ° C, 60% humidity (Table 2), 40 ° C, 75% humidity (Table 3). The finished pharma product were kept for 6 months to check the stability of the drug.

The average quality and stability of the finished products after the shelf life are shown in Table 2 and Table 3.

The above data demonstrate that the claimed combination of the drug in the described package can be stored for a specified shelf life of the drug - 2 years at a temperature of 25 ° C, without degradation of quality. Therefore, the claimed composition is stable during the study period.

The results obtained show that the claimed technical solution has a new, unknown from the prior art set of essential features. Accordingly, the claimed invention meets the requirements of patentability novelty and industrial applicability.

The claimed technical solution makes it possible to obtain a stable storage, transportation and convenient to use composition (solution) of edaravone, significantly extend the shelf life of the drug. Obtained according to the claimed invention, the drug retains its therapeutic properties, does not form impurities, is safe when opening the container with solution and use.

**Table 1. The results of the actual packaging of the compositions in different sets**

| Type of packaging | Quality characteristics of stability | | | | |
|---|---|---|---|---|---|
| | Time | pH | Accompanying impurities | Quantitative determination | Meets all requirements (yes/no) |
| | (storage) to study stability | From 3,0 to 4,5 | any impurity - not more 0,2% | Edaravone 0,285 - 0,315 (mg / ml) | |
| On release | 0 month | 4,2 | Less than 0,05% | 0,302 | yes |
| Polymeric package | 3 month | 4,1 | Less than 0,05% | 0,287 | yes |
| | 6 month | 3,9 | 0,06% | 0,277 | no |
| | 12 month | 3,8 | 0,13% | 0,271 | no |
| | 24 month | 3,6 | 0,24% | 0,263 | no |
| Bottle of borosilicate glass, sealed with a rubber cover | 3 month | 4,2 | Less than 0,05% | 0,293 | yes |
| | 6 month | 4,0 | Less than 0,05% | 0,287 | yes |
| | 12 month | 4,0 | Less than 0,05% | 0,281 | no |
| | 24 month | 3,9 | 0,08% | 0,278 | no |
| Plastic container manufactured by blow-fill-seal technology | 3 month | 4,1 | Less than 0,05% | 0,271 | no |
| | 6 month | 3,9 | 0,07% | 0,253 | no |
| | 12 month | 3,7 | 0,15% | 0,241 | no |
| | 24 month | 3,4 | 0,28% | 0,234 | no |
| Packaging according to the claimed technical solution | 3 month | 4,2 | Less than 0,05% | 0,300 | yes |
| | 6 month | 4,2 | Less than 0,05% | 0,299 | yes |
| | 12 month | 4,1 | Less than 0,05% | 0,298 | ves |
| | 24 month | 4,0 | Less than 0,05% | 0,298 | ves |

**Table 2. Results of the study the stability of the composition during storage at 25 ° C**

| Characteristics of quality (stability) | Appearance | Transp arency | The degree of color | Mechanical inclusions | pH | Osmol. | Accompanying impurities | Quantitative determinatio n | Sterility |
|---|---|---|---|---|---|---|---|---|---|
| QCT | Transparent colorless or light yellowish liquid. | Must be transpa rent | Not more intense for the standard Y₅ | Must stand the test | From 3,0 to 4,5 | From 275 to 325 mOsmol / kg | Any impurity - Not more than 0,2%. The amount of impurities - not more than 0,5% | Edaravone 0,285 - 0,315 (mg / ml) | Must be sterile |
| plugs type | | | | | | | | | |
| On release | Accord | Accord | Accord | Accord | 4,2 | 304 | Less than 0,05% | 0,302 | Accord |
| FEP | Accord | Accord | Accord | Accord | 4,1 | 301 | Less than 0,05% | 0,301 | Accord |
| PTFE | Accord | Accord | Accord | Accord | 4,2 | 297 | Less than 0,05% | 0,297 | Accord |
| PCTFE | Accord | Accord | Accord | Accord | 4,1 | 302 | Less than 0,05% | 0,299 | Accord |
| PFA | Accord | Accord | Accord | Accord | 4,0 | 306 | Less than 0,05% | 0,307 | Accord |
| ETFE | Accord | Accord | Accord | Accord | 4,3 | 301 | Less than 0,05% | 0,303 | Accord |
| PVDF | Accord | Accord | Accord | Accord | 4,0 | 298 | Less than 0,05% | 0,298 | Accord |
| PFPE | Accord | Accord | Accord | Accord | 4,1 | 302 | Less than 0,05% | 0,304 | Accord |

**Table 3. Results of the stability studies of the composition during storage at 40 ° C**

| Characteristics of quality (stability) | Appearance | Transp arency | The degree of color | Mechanical inclusions | pH | Osmol. | Accompanying impurities | Quantitative determinatio n | Sterility |
|---|---|---|---|---|---|---|---|---|---|
| QCT | Transparent colorless or light yellowish liquid. | Must be transpa rent | Not more intense for the standard Y₅ | Must stand the test | From 3,0 to 4,5 | From 275 to 325 mOsmol / kg | Any impurity - Not more than 0,2%. The amount of impurities - not more than 0,5% | Edaravone 0,285 - 0,315 (mg / ml) | Must be sterile |
| plugs type | | | | | | | | | |
| 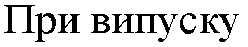 | Accord | Accord | Accord | Accord | 4,2 | 304 | Less than 0,05% | 0,302 | Accord |
| FEP | Accord | Accord | Accord | Accord | 3,9 | 301 | Less than 0,05% | 0,298 | Accord |
| PTFE | Accord | Accord | Accord | Accord | 3,8 | 303 | Less than 0,05% | 0,295 | Accord |
| PCTFE | Accord | Accord | Accord | Accord | 3,9 | 305 | Less than 0,05% | 0,299 | Accord |
| PFA | Accord | Accord | Accord | Accord | 3,7 | 301 | Less than 0,05% | 0,301 | Accord |
| ETFE | Accord | Accord | Accord | Accord | 3,8 | 297 | Less than 0,05% | 0,296 | Accord |
| PVDF | Accord | Accord | Accord | Accord | 3,9 | 295 | Less than 0,05% | 0,298 | Accord |
| PFPE | Accord | Accord | Accord | Accord | 4,0 | 304 | Less than 0,05% | 0,303 | Accord |

## Claims

1. A method of obtaining a liquid dosage form of an edaravone drug, stable during storage, transportation and convenient for use, for parenteral administration, said method provides:
i) preparing a solution containing as active ingredient edaravone or its pharmaceutically acceptable salts and excipients (acid component, alkaline component, antioxidant, osmolarizing agent and / or stabilizer);
ii) packing said dosage form into a pre-sterilized glass vial with a lid covered at least partially by an adhesive coating;
iii) closure of the vial with a lid at least partially covered with an anti-adhesive coating and sterilization the vial containing a solution of edaravone or its pharmaceutically acceptable salts as the active ingredient and excipients.

2. The method according to claim 1, wherein the glass vial is made of borosilicate glass.

3. The method according to claim 1, wherein the cover is made of a material of the class of elastic polymers.

4. The method according to claim 1, wherein the lid is coated inside or fully anti-adhesive coating of polychlorotrifluoroethylene (PCTFE), perfluoroalkoxy alkane (PFA), ethylene tetrafluoroethylene (ETFE), fluoroethylenepropylene (FEP), perfluoropolyether (PFPE) or polyvinyldene fluoride (PVDF).

5. A method of packing liquid dosage form of edaravone drug, that is stable during storage, transportation and convenient to use, for parenteral administration, said method involves: i) sterilization of a glass vial and a lid, ii) spill a solution containing edaravone or its pharmaceutically acceptable salts as the active ingredient and excipients (acid component, alkaline component, antioxidant, osmolarizing agent and/or stabilizer), into a sterilized glass vial; iii) closure of the vial containing solution with a lid covered at least partially by an anti-adhesive coating; iv) sterilization of the sealed vial containing liquid dosage form.

6. The method according to claim 5, wherein the glass vial is made of borosilicate glass.

7. The method according to claim 5, wherein the cover is made of rubber derivative or thermoplastic.

8. The method according to claim 5, wherein the cover inside or fully covered with an anti-adhesive coating of polychlorotrifluoroethylene (PCTFE), perfluoroalkoxy alkane (PFA), ethylene tetrafluoroethylene (ETFE), fluoroethylenepropylene (FEP), perfluoropolyether (PFPE) or polyvinyldene fluoride (PVDF).

9. A vial filled with a liquid dosage form of a medicinal product for parenteral administration containing as active ingredient edaravone or its pharmaceutically acceptable salts, and excipients (acid component, alkaline component, antioxidant, osmolarizing agent and/or stabilizer), and the vial is made of glass, covered by a lid made of a material based on elastic polymers, the lid is at least partially covered with an anti-adhesive coating.

10. The vial according to claim 9, made of borosilicate glass.

11. The vial according to claim 9, wherein the lid is made of rubber derivative or thermoplastic.

12. The vial according to claim 9, wherein the lid is covered inside or fully with an anti-adhesive coating of polychlorotrifluoroethylene (PCTFE), perfluoroalkoxy alkane (PFA), ethylene tetrafluoroethylene (ETFE), fluoroethylenepropylene (FEP), perfluoropolyether (PFPE) or polyvinyldene fluoride (PVDF).

13. The vial according to claim 9, wherein an aluminum cap that squeezes the lid of the vial to seal the container with the solution is used during packaging.
